# EUROPEAN PATENT APPLICATION

(11) **EP 1 902 697 A1**
(43) Date of publication of application: **26.03.2008**
(21) Application number: 07253558.6
(22) Date of filing: 07.09.2007
(51) Int. Cl.: A61F 13/49, A61F 13/56

(54) **An absorbent article and refastenable tabs therefor**

(30) Priority: 22.09.2006 US 526044
(71) Applicant: Tyco Healthcare Retail Services AG, 8201 Schaffhausen (CH)
(72) Inventor: Soto, Arginnys, Yardley, Pennsylvania 19067 (US); Erdman, Carol L., Chester, Pennsylvania 19380 (US)
(74) Representative: Elsy, David

(57) **Abstract**

Absorbent articles suitable for use as diapers or other hygiene products are provided. The absorbent articles include a pair of refastenable tabs that have a non-elastic portion and a fastener portion. The non-elastic portion has a top edge and a bottom edge that are asymmetrical to each other with respect to a horizontal axis. Products according to the invention may provide any of several benefits, including a high performance in terms of at least one of fit, comfort, producibility, and ease of use.

## Description

### FIELD OF THE INVENTION

The present invention relates to absorbent articles such as diapers, training pants, and the like. More specifically, the invention relates to absorbent articles with a refastening tab providing high performance in terms of at least one of fit, comfort, producibility, and ease of use.

### BACKGROUND OF THE INVENTION

Absorbent articles such as disposable diapers, training pants, and the like are known for their major function of absorbing and containing body exudates. Such articles are thus intended to prevent the soiling, wetting, or other contamination of clothing or other articles, such as bedding, that come in contact with the wearer. In the case of disposable diapers, they typically include a liquid permeable topsheet, a liquid impermeable backsheet, an absorbent core positioned between the topsheet and the backsheet, and a means for fastening the diaper about the wearer's waist.

The means for fastening has typically included tabs mounted on the front or rear section of the absorbent article. The tabs typically have included fasteners such as the hook or loop portion of a hook-and-loop fastener. While many developments have been made in the art of absorbent articles, there remains a need for further improvements in terms of at least one of fit, comfort, producibility, and ease of use.

### SUMMARY OF THE INVENTION

In one aspect, the invention provides an absorbent article having a chassis with a front section and a rear section. A pair of tabs are attached to either the front section or the rear section of the chassis and each of the tabs includes a non-elastic portion and a fastener portion and an optional elastic portion. The non-elastic portion has a top edge and a bottom edge that are asymmetrical with respect to each other along a horizontal axis. A fastener portion is attached to the non-elastic portion and is configured to engage either the front section or rear section of the chassis.

In another aspect of the invention, a method for producing an absorbent article having refastenable tabs is provided. The method includes the step of providing an absorbent article chassis having a front section and a rear section. A pair of non-elastic portions having a top edge and a bottom edge that are asymmetrical to each other with respect to a horizontal axis are also provided. The non-elastic portions are then directly or indirectly connected to the absorbent article chassis.

### BRIEF DESCRIPTION OF THE DRAWINGS

The features of the invention and the elements characteristic of the invention are set forth with particularity in the appended claims. The figures are for illustration purposes only and are not necessarily drawn to scale. The invention itself, however, may best be understood by reference to the detailed description which follows when taken in conjunction with the accompanying drawing in which:
FIG. 1 is a top plan view of an absorbent article according to one embodiment of the invention.
FIG. 2 is a top plan view of an absorbent article according to a second embodiment of the invention.
FIGS 3A, 3B, and 3C are top views of refastenable tabs according to exemplary embodiments of the invention.
FIG. 4 is a perspective view of an absorbent article or garment according to an embodiment of the present invention.

### DETAILED DESCRIPTION OF THE INVENTION

The invention will next be illustrated with reference to the figures. Such figures are intended to be illustrative rather than limiting and are included herewith to facilitate the explanation of exemplary features of embodiments of the present invention. The figures are not to scale, and are not intended to serve as engineering drawings.

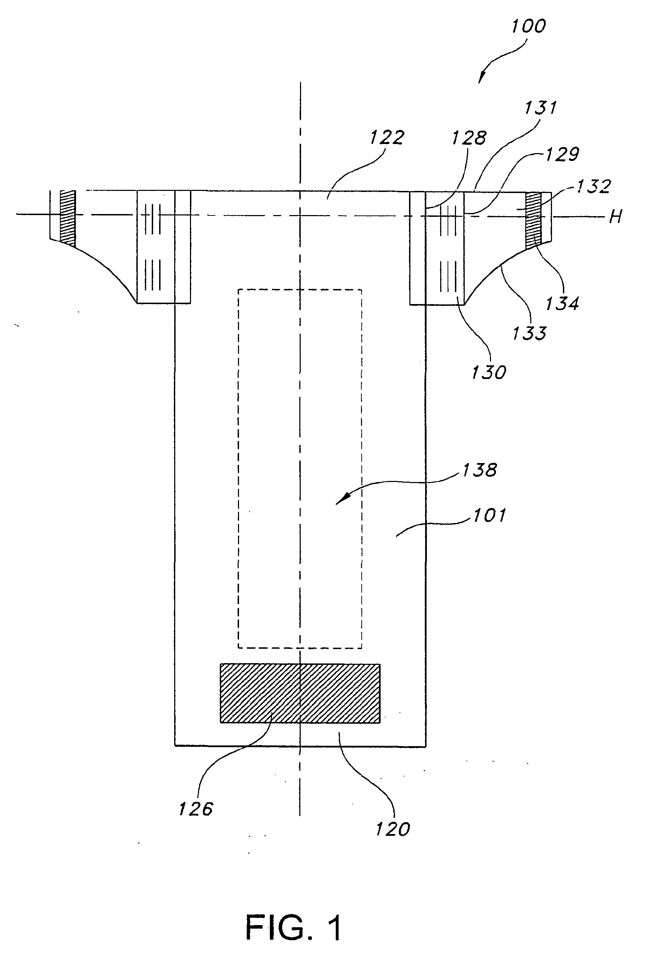

Referring generally to the drawings (FIGS. 1, 2, 3A, 3B, 3C, 4), the invention provides an absorbent article 100, 200, 400 having a chassis 101, 201, 301 with a front section 120, 220, 420 and a rear section 122, 222, 422. A pair of tabs are attached to either the front section or the rear section of the chassis and each of the tabs includes a non-elastic portion 132, 232, 332, 432, a fastener portion 134, 234, 334, 434, and an optionally excluded elastic portion 130, 230, 330, 430. The non-elastic portion has a top edge 131, 231, 331, 431 and a bottom edge 133, 233, 333, 433 that are asymmetrical to each other with respect to a horizontal axis. In an exemplary embodiment of an absorbent article including the elastic portion, the elastic portion is coupled to the chassis 101, 201, 301 along a. first connection 128, 228, 328, 428 and coupled to the non-elastic portion along a second connection 129, 229, 329, 429 which is at least as long as the first connection. The fastener portion 134, 234, 334, 434 is attached to the non-elastic portion and is configured to engage either the front section or rear section of the chassis.

In a further embodiment of the present invention, a method for producing an absorbent article 100, 200, 400 having refastenable tabs is provided. The method includes the step of providing an absorbent article chassis 101, 201, 301. A step is also provided for connecting a pair of elastic portions 130, 230, 330, 430 to a front 120, 220, 420 or rear 122, 222, 422 section of an absorbent article chassis 101, 201, 301 to form a first connection 128, 228, 328, 428. Non-elastic portions 132, 232, 332, 432 are connected to each elastic portion to form a second connection 129, 229, 329, 429 that is at least as long as the first connection. A fastener portion 134, 234, 334, 434 is attached to each non-elastic portion to complete the refastenable tabs on the absorbent article.

Referring now to the drawing, in which like reference numbers refer to like elements throughout the various figures that comprise the drawing, FIG. 1 depicts a top view of the absorbent article 100 according to one exemplary embodiment of the present invention. The absorbent article 100 is designed to contact the wearer along the waist and crotch areas and includes a substantially rectangular chassis 101 having a front section 120 and a rear section 122. The front section 120 is configured to be positioned against the anterior or front end of a wearer and the rear section 122 is configured to be positioned on the posterior or back end of a wearer. When worn by an individual standing upright, the chassis forms a lowest point on the absorbent article 100 when the front section 120 and the rear section 122 are positioned at the front and back of the individual, respectively.

The absorbent article 100 has a chassis 101 that typically includes an absorbent core 138 interposed between a barrier layer and a cover layer. When the absorbent article 100 is in place, the cover layer is arranged to face toward the body of the user (i.e. against the skin of the wearer) and the barrier layer is arranged facing away from the wearer. The cover layer is superimposed over the barrier layer, with the absorbent core 138 interposed in between. Although the cover layer and barrier layer are substantially rectangular in the depicted embodiment, the cover layer and/or barrier layer can be any suitable shape and dimension for a design of an absorbent article 100. The cover layer is joined to the barrier layer in any suitable manner such as by adhesive bonding, heat sealing, ultrasonic bonding, stitching or the like around at least the periphery of the absorbent core.

In order to enable liquid to quickly and efficiently pass through the cover layer and into the absorbent core 138 for trapping therein, the cover layer is preferably liquid permeable. In particular, the cover layer may be selected from a variety of textile-like films and fabrics. Suitable fabrics include non-woven materials that are pervious to liquid, soft and pliable. Preferred non-woven materials include spun-bonded polypropylene, spunbonded polyethylene, and thermally bonded webs of staple fibers, preferably polypropylene shape or sheath/core bi-component fibers having a core of polyester or polypropylene and a sheath of polyethylene. To enhance the fluid control properties of the aforementioned liners, surfactants or wetting agents typified by Triton^{®} X-100 and Triton^{®} X-102 available from Rohm & Haas Company of Philadelphia, PA may be applied to the fluid receiving zones of the liner selectively having the outer zones untreated to reduce migration of excreted fluid such as urine into the outer regions of the absorbent article, leading to leakage. If desired, the cover layer may be formed of a liquid impermeable material having plural apertures or pores extending therethrough so as to make the material liquid permeable.

As seen with respect to the embodiment of the absorbent article 100 shown in FIG. 1, the absorbent core 138 which is interposed between the cover layer and barrier layer may be of any shape, but is typically a rectangular member that is centered in the chassis 101 and extends from a portion of the front section 120 to a portion of the rear section 122. The absorbent core 138 may be made of any suitable absorbent material, as well as combinations of different types of absorbent materials. For example, the absorbent core 138 may be formed of a mixture of pulp fluff and superabsorbent polymer (SAP) wrapped in a liquid permeable tissue wrap (not shown). Examples of SAP include polyacrylamides, polyvinyl alcohol, polyacrylates, various grafted starches, and the like. A desired super absorbent material is a cross-linked polysodium acrylate, which can be purchased from BASF Corporation of Portsmouth, VA, under the trademark ASAP^{®} 2260. The super absorbent materials can be in various geometric forms, such as various shaped particles, fibers, foams, and layers. The fluff and SAP are present, for example, in a ratio such as about 11 grams of SAP to 16 grams of fluff for a size 4 diaper, and have a core density, for example, in a range such as about 0.14 to 0.22 grams per cubic centimeter. The amount of each absorbent material and SAP/fluff ratio depends on factors including the size of the article, e.g., "Small", "Medium", "Large" or "Extra Large."

The absorbent core 138 may be of any shape and may be a single, integral absorbent structure, or can comprise a plurality of individual separate absorbent structures and/or absorbent materials that are operably assembled together. It may also include an air-laid non-woven web that contains super-absorbent particles and/or super-absorbent fibers, polymeric binder and cellulose pulp fibers. In one exemplary embodiment, the absorbent core is sandwiched between two plies of tissue, is aligned on top of the barrier layer and adhered with construction adhesive. The tissue may typically have a basis weight of about 17 gsm. Suitable tissues are available from Cellu Tissue Corporation, East Hartford, CT. The absorbent core 138 is typically centered along the transverse direction of the absorbent article 100. In some embodiments of the invention, the front section 120 of the absorbent article 100 has a greater absorbent capacity than the rear section 122, for example by providing a greater basis weight of a superabsorbent material in the front vs. the rear section of the absorbent core 138.

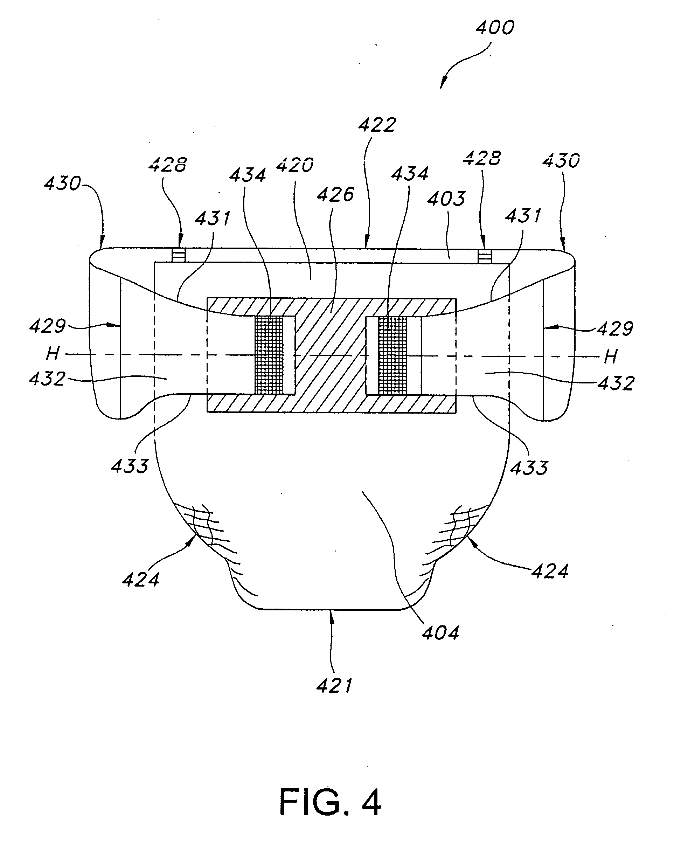

As further seen in FIG. 1. and later described, a landing zone 126 is attached to, or integral with, the barrier layer (such as barrier layer 404, FIG. 4) near the front section of the absorbent article 100. The barrier layer which is arranged facing away from the wearer (i.e. away from the skin of the wearer) is preferably formed from a laminated sheet of a non-woven material and film (with the non-woven side positioned as the outermost layer). Such material should preferably be hydrophobic, soft in texture, and strong in tensile strength. Suitable materials include a spunbond-meltblown-spunbond (SMS) web having a basis weight of about 10 to 20 grams per square meter (gsm), available from AVGOL Nonwoven Industries LTD., Holon, Israel, and a polypropylene film having a thickness of about 0.4 to 1.0 mils, available from Pliant Corporation, Williamsburg, VA. The spunbond layer is made of polypropylene fibers. Such composites provide the dual advantages of liquid barrier properties of film along with a soft, warm outer fabric texture. The non-woven outer cover can also be made of other suitable cloth-like materials, e.g., spun-bond or thermal-bond non-woven web made of either polypropylene, polyethylene, polyester, bi-component fibers (polyethylene/polypropylene or polyethylene/polyester), or any combinations of these fibers. Various multiple layer configurations or fiber denier variations may be used. Another example includes hydro-entangled non-woven webs, which may contain some cotton and/or rayon fibers blending in with thermal-plastic fibers. Cellulose fibers can also be blended in at small percentages to reduce cost. Still another example is a non-woven outer-cover made of stretchable or elastic materials, such as elastomeric composites of non-woven(s) and elastic membranes or a single layer of elastic material. The elastomeric composite can comprise an inner layer of pre-stretched extruded elastic film sandwiched between and attached to a pair of non-woven webs. The non-woven webs may consist of spun-bond web, thermal-bond web, or a combination of the two. Preferably, the elastic film is made of synthetic rubber and the non-woven made of spun-bond polypropylene.

Other materials for forming the barrier layer may include polyethylene films, polypropylene films, co-extruded films (polyethylene and ethylene vinyl acetate), co-polymer films (polyethylene/polypropylene), and polylaminates (polypropylene nonwoven and polyethylene film). Still another example is a film made of a "breathable" microporous polyethylene. Suitable breathable films are available from Tredegar Film Products, Richmond, VA. This material allows water vapor to pass through it over time, while being impervious to liquid water. The water vapor transmission rate may range from 200-2000 grams per square meter per 24-hour period.

With further reference to FIG. 1. the rear section 122 of the absorbent article 100 includes a pair of tabs extending away from the absorbent article 100 chassis. Each tab has a non-elastic portion 132, a fastener portion 134, and an optionally excluded elastic portion 130. The non elastic portion 132 has a top edge 131 and bottom edge 133 that are asymmetrical to each other with respect to a horizontal axis. In the exemplary embodiment, the elastic portion 130 is coupled to the chassis 101 along a first connection 128, and the non-elastic portion 132 is coupled to the elastic portion 130 along a second connection 129. The first and second connections may be achieved by an adhesive bond, an ultrasonic bond, a heat seal, stitching or a combination thereof or any other known coupling mechanism. Although the first connection 128 and second connection 129 are illustrated having substantially straight lines, it is contemplated that the first connection 128 and/or the second connection 129 may be curved or angled. By having a first connection 128 that is different than the second connection 129, forces on the tab acting upon the first connection 128 and second connection 129 may be differentiated. In another embodiment in which the elastic portion 130 is excluded from the tab, the non-elastic portion 132 is coupled to the chassis along the first connection 128 which may be straight, curved, angled, or a combination thereof.

The non-elastic portion 132 has a top edge 131 and a bottom edge 133 that are asymmetrical to each other with respect to a horizontal axis. In a preferred embodiment, the top edge 131 has a portion that is more horizontal than the bottom edge 133. It is contemplated that in this configuration, the non-elastic portion provides a better fit at the waist since tensile force is concentrated around the top edge 131 of the non-elastic portion when it is secured around the waist. The bottom edge 133 experiences lesser tensile force when the tab is secured, and provides a better fit and comfort area around the leg. In addition to the force gradient between the top edge 131 and bottom edge 133, force is also distributed along the first connection 128 and second connection 129. The non-elastic portion 132 may have at least one edge that is curved for comfort and fit which may contribute to additional coverage of the buttocks.

In other embodiments, the top edge 131 and bottom edge 133 each has a straight portion that is angled with respect to the horizontal axis. The angle of the straight portion on the top edge is different from the angle of the bottom edge. In general, the non-elastic portion 132 may be of any shape or dimension, but the top edge and bottom edge are asymmetrical when folded with respect to a horizontal axis. Thus, the non-elastic portion has at least one pair of edges that are non-parallel.

Each tab may optionally be formed from a substantially non-elastic material. The tabs may include a portion that is rendered elastic. For instance, an elastic portion can be provided by elasticizing an otherwise non-elastic portion of the tabs. This is optionally accomplished by introducing elastic strands or filaments into or adjacent the non-elastic material.

The non-elastic portion 132 may be formed from a single layer of non-woven material, and may exhibit substantially no elastic/stretch properties (i.e. inelastic). Further, non-elastic portion 132 may be formed of a different material than the absorbent article 100 chassis, thereby desirably allowing the use of less expensive, more breathable, and lower stiffness materials for non-elastic portion components than the chassis. The non-elastic portion 132 is optionally formed from a laminated sheet of a non-woven material and film (with the non-woven side positioned as the outermost layer). One particularly suitable material is a spunbond-meltblow-spunbond (SMS) web having a basis weight of about 5 to 60 gms per square meter (gsm), available from AVGOL Nonwoven Industries LTD., Holon, Israel. The spunbond layer is optionally made of polypropylene fibers. Such composites provide the dual advantages of liquid barrier properties of film along with a soft, warm outer fabric texture.

The non-elastic portion 132 can also be made of other suitable cloth-like materials, e.g., spun-bond or thermal-bond non-woven web made of either polypropylene, polyethylene, polyester, bi-component fibers (polyethylene/polypropylene or polyethylene/polyester), or any combinations of these fibers. Various multiple layer configurations or fiber denier variations may be used. Another example includes hydro-entangled non-woven webs, which may contain some cotton and/or rayon fibers blended in with thermal-plastic fibers. Cellulose fibers can also be blended in at small percentages to reduce cost. Still, other materials for forming the non-elastic portion 132 may include polypropylene films, co-extruded films (polyethylene and ethylene vinyl acetate), co-polymer films (polyethylene/polypropylene), and polylaminates (polypropylene nonwoven and polyethylene film). Yet another example is a film made of a "breathable" microporous polyethylene. Exemplary breathable films are available from Tredegar Film Products, Richmond, VA. This material allows water vapor to pass through it over time, while being impervious to liquid water. The water vapor transmission rate may range from 200-2000 grams per square meter per 24-hour period.

The non-elastic portion 132 is optionally selected from a variety of textile-like films and fabrics. Suitable fabrics include non-woven materials that are impervious to liquid, soft and pliable. Exemplary non-woven materials include spunbonded polypropylene, spunbonded polyethylene, and thermally bonded webs of staple fibers, preferably sheath/core bi-component fibers having a core of polyester or polypropylene and a sheath of polyethylene.

As depicted in FIG. 1, each non-elastic portion 132 also includes a fastener portion 134. The fastener portion 134 may include a hook assembly of a hook-and-loop fastener, such as are known in the art that may be non-permanently attached to a loop assembly on the landing zone 126. The fastener portion 134 may be any known releasable fastener, including adhesives, co-adhesives, tapes, buttons, and other fastening mechanisms used in the art with the complementary fastener being a receptive area on the landing zone 126. It has been discovered that fastener portion 134 providing material properties such as compression modulus and stiffness within certain ranges are particularly suitable for making absorbent articles with a high level of comfort for the wearer, as well as ease of donning and doffing the product without tearing of the garment or, instead, inadvertent disengagement of the fasteners. Thus the absorbent article of this invention has fastener portions that are re-fastenable. The non-elastic portion 132 may further include a supplemental tab component or portion (not shown) to allow easy detachment of hook (or fastening tape) assembly from loop (or frontal tape), or, in general, fastener portion 134 from the front section 120 of the absorbent article 100. The fastener portion 134, furthermore, may comprise an inelastic material and may further include a finger tab at one end, or disposed along its perimeter.

As further seen in the exemplary embodiment shown in FIG. 1, the elastic portion 130 is coupled to the absorbent article chassis along a first connection 128 and coupled to the non-elastic portion 132 along a second connection 129. The second connection 129 is at least as long as the first connection 128 and in this configuration it is contemplated that when the non-elastic portion is pulled to stretch the elastic portion in at least one direction (such as the direction corresponding to the longitudinal axis of the tab and outward to the side of the absorbent article), force is distributed along the length of the second connection 129 and the first connection 128. This prevents de-coupling of the non-elastic portion 132 from the elastic portion 130 or de-coupling of the elastic portion 130 from the absorbent article chassis 101. It is further contemplated that, by distributing force along the second and first connections, any distortion of the tabs when they are stretched during use can be reduced. This is believed to result from the fact that the tension applied to the tabs when they are pulled outwardly from the side of the absorbent article is distributed over the same length of connection in this embodiment as opposed to being concentrated along a shorter length for one connection than the other.

An embodiment in which the second connection 129 is at least as long as the first connection 128 also allows full utilization of the elastic material that forms the elastic portion 130 between the non-elastic portion 132 and chassis 101. In other words, it is believed that, to the extent one connection is longer or otherwise larger than the other, the geometric portion of the elastic material that corresponds to that difference in connection size is not fully utilized when the tab is pulled or otherwise placed in tension. Although the second connection 129 has been particularly illustrated and described as having a length which is at least as long as the first connection 128, in other embodiments, the first connection 128 may have a length at least as long as the second connection 129.

The elastic portion 130 is preferably stretchable in at least two directions and has a first shape that is substantially rectangular in an unstretched configuration, and a second shape in a stretched configuration. By having an elastic portion 130 that has substantially straight edges, high manufacturing costs are avoided and less elastic material is wasted. It is also contemplated that an elastic portion 130 with at least a pair of edges that have equal lengths and are paraiiei with respect to each other have a more even spread of force per unit area as the elastic portion 130 is stretched in at least one direction.

As mentioned previously, this feature of this embodiment prevents de-coupling of the non-elastic portion 132 from the elastic portion 130 and decreases the force applied per unit area on the second connection 129 as the elastic portion 130 is stretched. Other benefits of this particular embodiment include distributing force more evenly along the second and first connection which reduces distortion of the tabs when stretched and allows full utilization of the elastic portion 130 between the non-elastic portion 132 and chassis 101. In FIG. 1, the first connection 128 and second connection 129 are oriented substantially parallel to each other, but in other embodiments the first connection 128 or the second connection 129 may be non-parallel or curved. The non-elastic portion 132 can also be configured such that at least a pair of edges on the non-elastic portion 132 are non-parallel.

The elastic portion 130 may be formed from a fluted elastic or stretch non-woven laminate material obtained, for example, from Tredegar Film Products of Richmond, VA. The elastic portion 130 may include a high-stretch laminate comprising an elastic middle layer sandwiched in between two cloth-like materials. Other stretchable or elastic materials may be used such as elastomeric composites of non-woven(s) and elastic membranes or a single layer of elastic material. A suitable elastomeric composite can comprise an inner layer of pre-stretched extruded elastic film sandwiched between and attached to a pair of non-woven webs. The non-woven webs may consist of spun-bond web, thermal-bond web, or a combination of the two. Optionally, the elastic film is made of synthetic rubber and the non-woven made of spun-bond polypropylene. Other elastic and non-elastic materials may be used as well, depending on design considerations.

In some embodiments of the invention, the elastic portion 130 comprises an elastic material applied in a stretched configuration to a non-elastic substrate, which may for example be a polyolefin or other material used to form a barrier layer for the absorbent article 100. Exemplary elastic materials include elastic films, foams, and nonwoven materials, suitable examples and methods of application of which are known in the art. Examples include Fabriflex^{®} stretch nonwoven film laminate, available from Tredegar Corporation of Richmond, VA, or polyurethane foam laminate, available from General Foam Corporation of Paramus, NJ. The elastic portion 130 which is coupled to the chassis 101 of the absorbent absorbent article 100 may comprise any of a number of elastic materials known in the art, with typical exemplary materials being high-stretch laminates having an elastic middle layer sandwiched between two cloth-like outer layers, such as Fabriflex^{®} film laminate.

A landing zone 126 suitable for engagement with the fastener portion 134 is located on the front section 120 of the absorbent article chassis 101. The area in which the fastener portions 134 and landing zone 126 contact each other will be referred to herein as "engageable portions" of these features. Typically the fastener portion 134 will be of a smaller size than landing zone 126, and hence essentially the entirety of fastener portion 134 will be engageable, while only that portion of landing zone 126 that contacts fastener portion 134 is engaged. However, other configurations are contemplated as well.

The landing zone 126 is preferably located on a surface of the absorbent article chassis 101 opposite that bearing the elastic portion 130, non-elastic portion, and fastener portion 134, so that fastener portion 134 and landing zone 126 may engage each other when the absorbent article 100 is worn by a user. The landing zone 126 is typically of a rectangular shape, but any shape may be used. There may be a single landing zone configured to receive both fasteners portions 134, or separate landing zones may be provided for each fastener portion. Although this embodiment of the invention in FIG. 1 shows a pair of tabs as being mounted on the rear section 122, and the landing zone 126 on the front section 120, these positions may be reversed. It has been discovered that a product that attaches to a landing zone in the rear section 122 may be of particular benefit in the toilet training of children, by helping to avoid tampering by the child. Further, rear closure may reduce the potential for scratching or other skin irritation by the components of the closure system, for example when the wearer bends forward at the waist to sit or stoop.

Although not shown in FIG. 1, elastic leg gathers may be included so that they extend along the leg opening region of the absorbent article, as disclosed in U.S. Pat. No. 4,695,278 to Lawson and U.S. Pat. No. 4,795,454 to Dragoo, which are incorporated herein by reference. Each gather is elasticized and extends along the side marginal edges of the absorbent article 100 chassis.

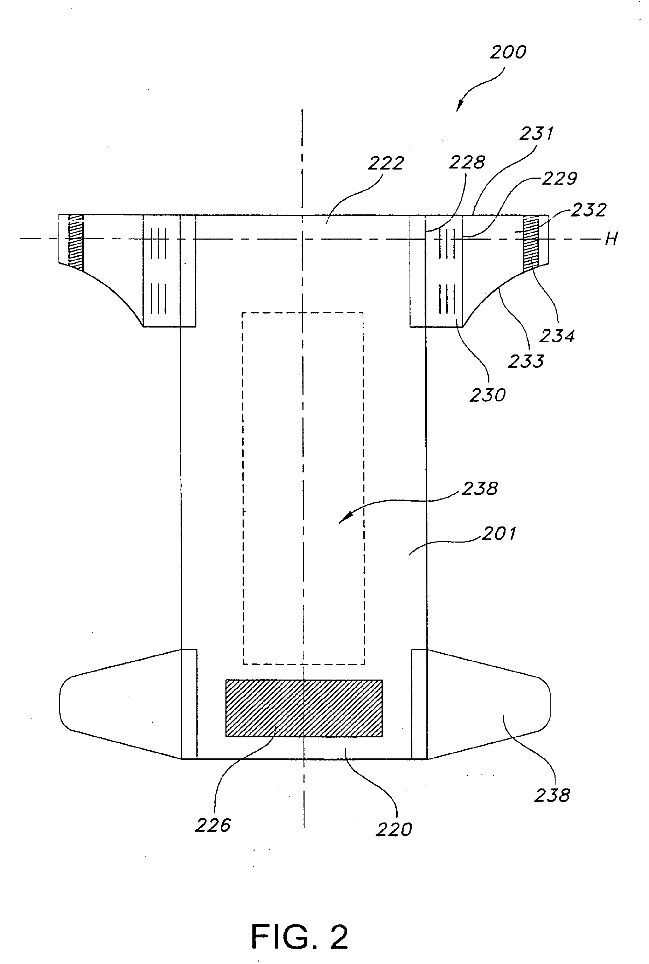

FIG. 2 shows an alternative embodiment of the absorbent article 100 as described above in FIG. 1. The absorbent article 200 includes a chassis 201 having a front section 220 and a rear section 222. An absorbent core is 238 is interposed between a barrier layer (such as barrier layer 404, FIG. 4) and cover layer (such as cover layer 403, FIG. 4) on absorbent article 200. The rear section 222 of the absorbent article 200 includes a pair of tabs having a non-elastic portion 232, a fastener portion 234, and an optionally excluded elastic portion 230. The elastic portion 230 is attached to the chassis along a first connection 228, and the non-elastic portion 232 is attached to the elastic portion 230 along a second connection 229 that is at least as long as the first connection 228. A pair of extensions 238 are coupled to the front section of the absorbent article's chassis to facilitate removal of the absorbent article. In another embodiment, the extensions can be coupled along the rear portion 222 of the absorbent article's chassis. The extensions 238 can be of any shape or dimension coupled to the absorbent article's chassis and may be formed from any substantially non-elastic material as previously described in FIG. 1.

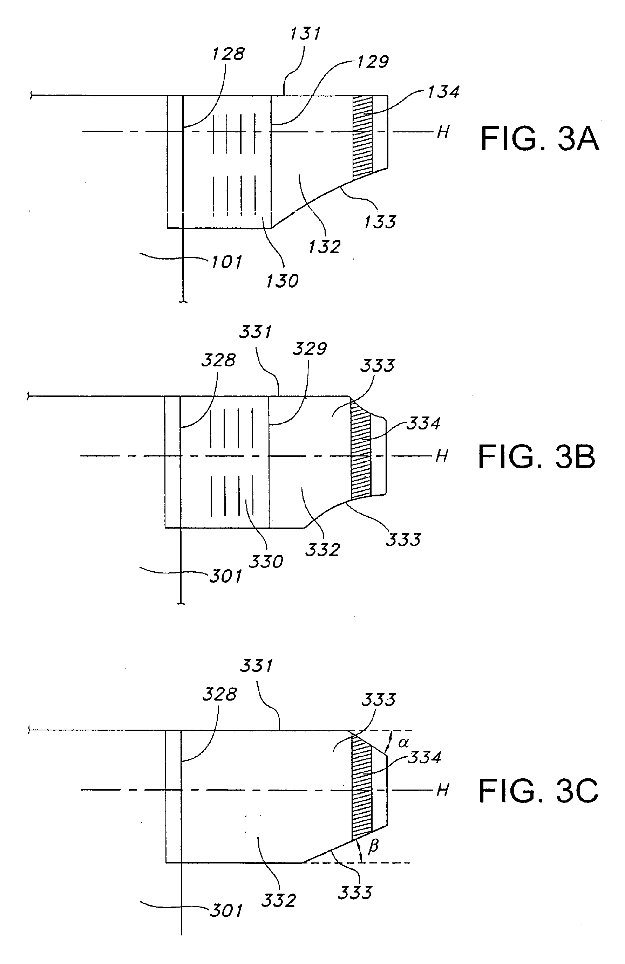

FIGS. 3A,3B, and 3C show top views of refastenable tabs according to other exemplary embodiments of the invention. With respect to FIG. 3A & 3B, the tabs include an elastic portion 130, 330 coupled to an absorbent article chassis along a first connection 128, 328 and a non-elastic portion 132, 332 coupled to the elastic portion along a second connection 129, 329. The edge that forms the second connection 129, 329 is at least as long as the first connection 128, 328 in order to prevent de-coupling of the non-elastic portion 132, 332 from the elastic portion 130, 330. This also decreases the force applied per unit area on the second connection 129, 329 as the elastic portion 130, 330 is stretched. The non-elastic portion 132, 332, as previously described in FIG. 1, may be of any shape or dimension, but generally has a top edge 131, 331 and a bottom edge 133, 333 that are asymmetrical to each other with respect to a horizontal axis. The non-elastic portion 134, 334 may have at least one edge 331, 333 which is curved to provide comfort and fit around a leg or waist area. A fastener portion 134, 334 is attached to the non-elastic portion to engage a landing zone on the absorbent article.

FIG. 3C shows a top view of a refastenable tab without an elastic portion. The non-elastic portion 332 is coupled to the absorbent article chassis along a first connection 328. The non-elastic portion has a top edge and a bottom edge that each has a straight portion angled with respect to a horizontal axis. The angle of the straight portion on the top edge is different from the angle of the straight portion on the bottom edge, such that if the top and bottom edge were folded along the horizontal axis, the top and bottom edge would be asymmetrical.

FIG. 4 is a perspective view of an absorbent article 400 or garment according to another embodiment of the present invention. In the embodiment shown, the elastic portion 430 is coupled to a rear section 422 of the absorbent article chassis along a first connection 428. A non-elastic portion 432 is coupled to the elastic portion 430 by a second connection 429. The second connection 429 has a length at least as long as the first connection 428. A fastener portion 434 is attached to the non-elastic portion 432 (viewed here through the surface of the non-elastic portion 432). The absorbent article is depicted in FIG. 4 with fastener portions 434 engaged to landing zone 426 on the front section 420. It is noted that the landing zone 426 may be positioned on the rear section 422 of the chassis and the tabs on the front section 420 to engage the fastener portion 434 to the rear section 422 of the chassis. When worn by an individual, the absorbent article forms a lowest point 421. A cover layer 403 is positioned against the skin of a wearer and an absorbent core is interposed between the cover layer 403 and a barrier layer 404. Elastic leg gathers 424 are located along the sides of the absorbent article chassis, but can be optionally excluded in other embodiments.

Although the present invention has been particularly described in conjunction with specific embodiments, it is evident that many alternatives, modifications, and variations will be apparent to those skilled in the art. It is therefore contemplated that the appended claims will embrace any such alternatives, modifications, and variations as falling within the true scope and spirit of the present invention.

## Claims

1. An absorbent article comprising:
a chassis having a front section and a rear section, the front section or the rear section of the chassis having a pair of tabs extending therefrom, each tab comprising:
a non-elastic portion coupled to the chassis having a top edge and a bottom edge that are asymmetrical to each other with respect to a horizontal axis; and
a fastener portion attached to the non-elastic portion, the fastener portion being configured to engage the front section or the rear section of the chassis.

2. The absorbent article according to claim 1, wherein the top edge and the bottom edge each has a straight portion angled with respect to the horizontal axis, the angle of the straight portion on the top edge being different from the angle of the straight portion on the bottom edge.

3. The absorbent article according to claim 1, wherein the non-elastic portion has at least one edge that is curved.

4. The absorbent article according to claim 1, wherein the non-elastic portion has at least one pair of edges that are non-parallel.

5. The absorbent article according to claim 1, wherein each tab further comprises an elastic portion coupled to the chassis along a first connection and coupled to the non-elastic portion along a second connection.

6. The absorbent article according to claim 5, wherein the second connection is at least as long as the first connection.

7. The absorbent article according to claim 5, wherein the first connection is at least as long as the second connection.

8. The absorbent article according to claim 5, wherein the first connection and second connection are oriented substantially parallel to each other.

9. The absorbent article according to claim 5, wherein the first connection and second connection are oriented substantially non-parallel to each other.

10. The absorbent article according to claim 5, wherein the first connection or second connection is curved or angled.

11. The absorbent article according to claim 5, wherein the elastic portion is stretchable in at least one direction.

12. The absorbent article according to claim 5, wherein the elastic portion is stretchable in at least two directions.

13. The absorbent article according to claim 5, wherein the elastic portion is formed from a film, a foam, or a non-woven material.

14. The absorbent article according to claim 5, wherein the elastic portion is substantially rectangular.

15. The absorbent article according to claim 5, wherein the elastic portion has at least one pair of edges that have equal lengths.

16. The absorbent article according to claim 5, wherein the elastic portion has at least one pair of edges that are parallel to each other.

17. The absorbent article according to claim 5, wherein the elastic portion has substantially straight edges.

18. An absorbent article comprising:
a chassis having a front section and a rear section; and
a pair of tabs extending from the front section or the rear section of the chassis, each tab comprising an elastic portion and a non-elastic portion, said non-elastic portions of said tabs being asymmetrical with respect to a horizontal axis.

19. The absorbent article according to claim 18, wherein the elastic portion is provided by elasticizing an otherwise non-elastic portion of each tab.

20. The absorbent article according to claim 1, wherein the chassis has a substantially rectangular configuration.

21. The absorbent article according to claim 1, wherein the front section or the rear section of the chassis comprises a pair of extensions.

22. The absorbent article according to claim 1, wherein the front section or the rear section of the chassis comprises a pair of extensions that are substantially non-elastic.

23. The absorbent article according to claim 1, wherein the fastener portion is refastenable to the front section or the rear section of the chassis.

24. The absorbent article according to claim 1, wherein the fastener portion is selected from the group consisting of an adhesive, a co-adhesive, a button, a latch, hooks, loops, and a snap.

25. The absorbent article according to claim 1, wherein the front section or the rear section of the chassis includes a landing zone to engage the fastener portion.

26. The absorbent article according to claim 25, wherein the landing zone is selected from the group consisting of an adhesive, a co-adhesive, a button, a latch, hooks, loops, and a snap.

27. A method for producing an absorbent article, the method comprising the steps of:
providing an absorbent article chassis having a front section and a rear section;
providing a pair of non-elastic portions having a top edge and a bottom edge that are asymmetrical to each other with respect to a horizontal axis, each of the non-elastic portions further including a fasterner portion attached to the non-elastic portion, the fastener portion being configured to engage the front section or the rear section of the chassis; and
connecting the non-elastic portions to the absorbent article chassis.

28. The method according to claim 27, wherein said providing step includes forming a top edge and a bottom edge of the non-elastic portion along an angle with repect to a horizontal axis, the angle of the top edge being different from the angle of the bottom edge.

29. The method according to claim 27, wherein at least one of said connecting steps comprises adhesive bonding, ultrasonic bonding, heat sealing, or stitching.

30. The method according to claim 27,wherein the connecting step further comprises connecting a pair of elastic portions to the absorbent article chassis along a first connection.

31. The method according to claim 30, wherein said connecting step further comprises the step of connecting the non-elastic portions to the elastic portions along a second connection at least as long as the first connection.

32. The method according to claim 31, wherein said connecting step is preformed to orient the first and second connection substantially parallel to each other.

33. The method according to claim 27, wherein said connecting step comprises connecting the non-elastic portions directly to the absorbent article chassis.

34. The absorbent article according to claim 1, wherein the non-elastic portion is coupled directly to the chassis.

35. The absorbent article according to claim 1, wherein the non-elastic portion is coupled indirectly to the chassis.

36. The absorbent article according to claim 1, wherein the bottom edge of the non-elastic portion includes a convex portion configured to provide additional coverage of the buttock region of a wearer.
